# EUROPÄISCHE PATENTSCHRIFT

(11) **EP 1 005 462 B1**
(45) Veröffentlichungstag und Bekanntmachung des Hinweises auf die Patenterteilung: **29.11.2006**
(21) Anmeldenummer: 98942624.2
(22) Anmeldetag: 25.07.1998
(51) Int. Cl.: C07D 285/16

(54) **VERFAHREN ZUR HERSTELLUNG VON 3-ISOPROPYL-1H-2, 1,3-BENZOTHIADIAZIN-4 (3H)-ON-2,2-DIOXID**
METHOD FOR THE PRODUCTION OF 3-ISOPROPYL-IH-2, 1,3-BENZOTHIADIAZINE-4 (3H)-ONE-2,2-DIOXIDE
PROCEDE POUR LA PREPARATION DE 3-ISOPROPYL-1H-2, 1,3-BENZOTHIADIAZIN-4 (3H)-ONE-2,2-DIOXYDE

(30) Priorität: 19.08.1997 DE 19735682
(43) Veröffentlichungstag der Anmeldung: 07.06.2000
(73) Patentinhaber: BASF Aktiengesellschaft, 67056 Ludwigshafen (DE)
(72) Erfinder: MERKLE, Hans, Rupert, D-67061 Ludwigshafen (DE); WÖRZ, Otto, D-67159 Friedelsheim (DE); FRETSCHNER, Erich, D-69239 Neckarsteinach (DE); HANSEN, Hanspeter, D-67061 Ludwigshafen (DE); MÜLLER, Albrecht, D-67071 Ludwigshafen (DE); BENZ, Kurt, D-67354 Römerberg (DE)
(86) Internationale Anmeldenummer: PCT/EP1998/004664
(87) Internationale Veröffentlichungsnummer: WO 1999/009019

(56) Entgegenhaltungen:
- DE-A- 2 710 382
- CHEMICAL ABSTRACTS, vol. 118, no. 15, 12. April 1993 Columbus, Ohio, US; abstract no. 147565b, Seite 844; XP002084457 & CN 1 063 688 A (FAMING ZHUANLI SHENQING GONGKAI SHUOMINGSHU) 19. August 1992
- CHEMICAL ABSTRACTS, vol. 117, no. 9, 31. August 1992 Columbus, Ohio, US; abstract no. 90332w, Seite 788; XP002084458 & CZ 270 529 A (M. POLIEVKA ET AL.) 25. Juni 1991

## Beschreibung

Die vorliegende Erfindung betrifft ein neues Verfahren zur Herstellung von 3-Isopropyl-1H-2,1,3-benzothiadiazin-4(3H)-on-2,2-dioxid (I) oder eines Salzes von I 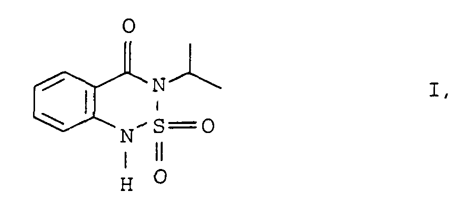 indem man gleichzeitig Anthranilsäureisopropylamid II 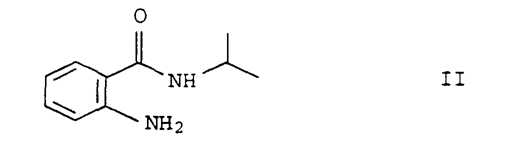 mit Schwefeltrioxid oder Chlorsulfonsäure in Gegenwart einer organischen Base oder mit Addukten von Schwefeltrioxid an organischen Basen und mit Phosphoroxychlorid bei 50°C bis Rückflußtemperatur umsetzt und gewünschtenfalls anschließend in ihre Salze überführt.

Es ist bekannt, daß 2,1,3-Benzothiadiazin-4-on-2,2-dioxid-Derivate erhalten werden, indem man Anthranilsäureamid-Derivate mit Schwefeltrioxid-Derivaten in Gegenwart einer organischen Base bei 0°C bis Raumtemperatur zu den entsprechenden Sulfamsäure-Salzen umsetzt und anschließend die Cyclisierung dieser Salze durchführt (DE-A 27 10 382).

Da bei diesem Verfahren jedoch Salze bzw. Suspensionen gehandhabt werden müssen, wird die großtechnische Herstellung der Verbindung I oder seiner Salze erschwert.
Auch die Reinheit der so erhaltenen Verbindungen I oder seiner Salze ist nicht befriedigend.
Weiterhin ist bekannt, daß die Sulfamsäure-Salze bei erhöhter Temperatur nicht stabil sind.

Der vorliegenden Erfindung lag daher die Aufgabe zugrunde, ein einfaches, kostengünstiges und großtechnisch anwendbares Verfahren zur Herstellung der Verbindung I oder seiner Salze zu finden, das befriedigende Produktreinheiten liefert.

Demgemäß wurde ein Verfahren zur Herstellung von 3-Isopropyl-1H-2,1,3-benzothiadiazin-4(3H)-on-2,2-dioxid (I) gefunden, indem man die Edukte in Gegenwart einer Base gleichzeitig bei 50°C bis Rückflußtemperatur umsetzt.

Die Umsetzung kann für den Fall der Verwendung von 2-Picolin als Base durch folgende Reaktionsgleichung wiedergegeben werden

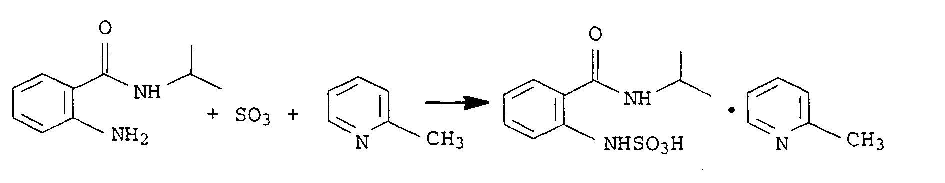

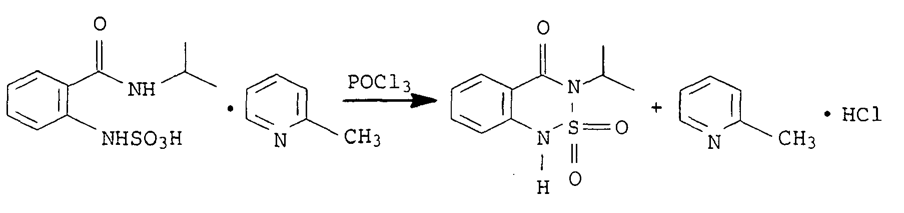

Das erfindungsgemäße Verfahren zeichnet sich dadurch aus, daß in einer Stufe das Sulfamsäure-Salz bei 50°C bis Rückflußtemperatur in situ gebildet wird und sofort mit dem bereits vorliegenden Phosphoroxychlorid zu Verbindung I cyclisiert wird.

Die Umsetzung kann in Substanz oder in Lösung durchgeführt werden. Als Lösungsmittel kommen inerte organische Lösungsmittel, z.B. aliphatische Kohlenwasserstoffe wie Pentan, Hexan, Heptan oder Octan, halogenierte aliphatische Kohlenwasserstoffe wie Methylenchlorid, Chloroform, Tetrachlorkohlenstoff, 1,2-Dichlorethan oder Dichlorpropane, halogenierte aromatische Kohlenwasserstoffe wie Chlorbenzol oder Dichlorbenzole, Ether wie Diethylether oder Methyl-tert.-butylether, Amide wie Dimethylformamid oder Gemische hiervon in Betracht.
Bevorzugt wird die Umsetzung in Lösung, insbesondere in einem halogenierten organischen Lösungsmittel, also einem halogenierten aliphatischen oder aromatischen Lösungsmittel durchgeführt. Vorzugsweise verwendet man halogenierte, aliphatische Lösungsmittel, insbesondere 1,2-Dichlorethan.

Bei dem erfindungsgemäßen Verfahren können beispielsweise folgende organische Basen verwendet werden: Trialkylamine wie Trimethylamin, Triethylamin, Dimethylethylamin, Dimethylpropylamine, Dimethylbutylamine, Dimethylcyclohexylamin oder Tributylamin; N-Methylmorpholin, N-Ethylmorpholin oder N-Methylpiperidin; N,N-Dialkylaniline wie Dimethylanilin, Diethylanilin, Methylethylanilin, N,N-Dialkylamide wie Dimethylformamid oder Dimethylacetamid; Tetraalkylharnstoffe, z.B. Tetramethylharnstoff oder Tetraethylharnstoff; oder aromatische organische Base wie Pyridin, substituiertes Pyridin, z.B. 2-Picolin, 3-Picolin oder 4-Picolin, Chinolin, Lutidin, Chinaldin oder Gemische hiervon.

Vorzugsweise werden als Basen aromatische organische Basen, insbesondere Pyridin oder substituierte Pyridine verwendet. Außerordentlich bevorzugt wird 2-Picolin verwendet.

Die Umsetzung wird zweckmäßigerweise so durchgeführt, daß man 2,0 bis 1,0 mol, vorzugsweise 1,4 bis 1,1 mol Schwefeltrioxid und 4,0 bis 1,6 mol einer der oben angeführten Basen in einem unter den Verfahrensbedingungen inerten Lösungsmittel wie oben genannt zu dem Schwefeltrioxidaddukt umsetzt. Es ist aber auch möglich, separat hergestelltes Schwefeltrioxidaddukt in einem unter den Reaktionsbedingungen inerten Lösungsmittel, ggf. unter Zugabe der entsprechenden Base, zu lösen und diese Lösung in der weiteren Reaktion einzusetzen. Zu der Lösung des Schwefeltrioxidaddukts gibt man gleichzeitig bei 50°C bis Rückflußtemperatur, vorzugsweise bei 65°C bis 85°C, 1 mol Anthranilsäureisopropylamid II in Lösung oder in Substanz und 2,0 bis 0,3 mol, insbesondere 1,2 bis 0,5 mol Phosphoroxychlorid zu. Man rührt diese Mischung bei einer Temperatur von 50°C bis Rückflußtemperatur, vorzugsweise bei 65 bis 85°C, für 30 min bis 6 h, insbesondere für 30 min bis 4 h. Anschließend wird das Reaktionsgemisch bei oben genannter Reaktionstemperatur oder nach Abkühlen mit Wasser hydrolysiert und aufgearbeitet. Hierbei wird die organische Phase abgetrennt, diese mit Wasser gewaschen und mit einer wäßrigen Base, z.B. anorganischen Basen, wie aus Natriumhydroxid, Kaliumhydroxid, Magnesiumhydroxid, Ammoniumhydroxid, Natriumcarbonat, Kaliumcarbonat, Magnesiumcarbonat, Natriumhydrogencarbonat, Kaliumhydrogencarbonat etc. und Wasser oder z.B. organische Basen wie Dimethylamin, Trimethylamin oder Diethanolamin etc., extrahiert. Diese durch Extraktion erhaltene wäßrige Salzlösung von I kann noch Lösungsmittel enthalten, das destillativ entfernt werden kann. Die resultierende Salzlösung von I kann nun zu anwendungsorientierten Darreichungsformen weiterverarbeitet werden.

Es ist aber auch möglich, von der durch Extraktion erhaltenen wäßrigen Salzlösung von I Lösungsmittelreste und das Wasser vollständig zu entfernen und das so erhaltene Salz von I zu den anwendungsorientierten Darreichungsformen weiterzuverarbeiten.

Weiterhin ist es möglich, die durch Extraktion erhaltene wäßrige Salzlösung von I anzusäuern, z.B. mit Salzsäure, Schwefelsäure oder Phosphorsäure. Der sich bildende Niederschlag von I wird dann abgesaugt, gegebenenfalls gewaschen und getrocknet. So erhaltene Verbindung I kann nun zu den anwendungstechnischen Darreichungsformen weiterverarbeitet werden.

Bevorzugt werden 2,0 bis 1,0 mol, insbesondere 1,4 bis 1,1 mol, Schwefeltrioxid und 4,0 bis 1,6 mol 2-Picolin in 1,2-Dichlorethan zu dem Schwefeltrioxidaddukt umgesetzt. Zu dieser Lösung gibt man gleichzeitig bei 50°C bis Rückflußtemperatur, insbesondere bei 65°C bis 85°C, 1 mol Anthranilsäureisopropylamid II in 1,2-Dichlorethan und 2,0 bis 0,3 mol, insbesondere 1,2 bis 0,5 mol, Phosphoroxychlorid zu. Man rührt diese Mischung vorzugsweise bei 65°C bis 85°C für 30 min bis 6 h, insbesondere 30 min bis 4 h. Anschließend wird wie voranstehend erläutert zum Produkt hin aufgearbeitet.

Ebenso kann die Umsetzung zweckmäßigerweise so durchgeführt werden, daß man 2,0 bis 1,0 mol, vorzugsweise 1,4 bis 1,2 mol, Schwefeltrioxid, 4,0 bis 1,6 mol einer der oben aufgeführten Basen, 1 mol Anthranilsäurepropylamid II und 2,0 bis 0,3 mol, insbesondere 1,2 bis 0,5 mol Phosphoroxychlorid gleichzeitig in einem unter den Verfahrensbedingungen inerten Lösungsmittel, wie oben genannt, bei 50°C bis Rückflußtemperatur, vorzugsweise bei 65°C bis 85°C, umsetzt. Man rührt diese Mischung bei einer Temperatur von 50°C bis Rückflußtemperatur, vorzugsweise bei 65 bis 85°C, für 30 min bis 6 h, insbesondere für 30 min bis 4 h. Anschließend wird, wie voranstehend erläutert, zum Produkt hin aufgearbeitet.

Bevorzugt werden 2,0 bis 1,0 mol, insbesondere 1,4 bis 1,1 mol, Schwefeltrioxid, 4,0 bis 1,6 mol 2-Picolin in 1,2-Dichlorethan, 1 mol Anthranilsäureisopropylamid II in 1,2-Dichlorethan und 2,0 bis 0,3 mol, insbesondere 1,2 bis 0,5 mol Phosphoroxychlorid gleichzeitig bei 50°C bis Rückflußtemperatur, insbesondere bei 65°C bis 85°C, umgesetzt. Man rührt diese Mischung bei einer Temperatur von 50°C bis Rückflußtemperatur, vorzugsweise bei 65 bis 85°C, für 30 min bis 6 h, insbesondere für 30 min bis 4 h. Anschließend wird, wie voranstehend erläutert, zum Produkt hin aufgearbeitet.

Bei der Umsetzung wird in der Regel das Verhältnis Base, insbesondere 2-Picolin, und Schwefeltrioxid so gewählt, daß dieses etwa 2:1 beträgt.

Es ist aber auch möglich, anstelle von Schwefeltrioxid Chlorsulfonsäure zu verwenden. In diesem Fall beträgt das Verhältnis Base, insbesondere 2-Picolin, zu Chlorsulfonsäure etwa 3:1.

Somit resultiert als vorteilhaftes Verhältnis von gebildetem Schwefeltrioxid-Addukt und organischer Base, insbesondere 2-Picolin, von etwa 1:1.

In der Regel ist die Lösung von Base in organischem Lösungsmittel, wie z.B. 2-Picolin in 1,2-Dichlorethan, 30 bis 10%ig, vorzugsweise 25 bis 15%ig.

Weiterhin ist die Lösung von Anthranilsäureisopropylamid II in organischem Lösungsmittel, wie 1,2-Dichlorethan, in der Regel 25 bis 5%ig, vorzugsweise 20 bis 8%ig.

Die verwendete Base, wie 2-Picolin, und das verwendete Lösungsmittel, wie 1,2-Dichlorethan, können zurückgewonnen werden und von neuem in das Verfahren eingeschleust werden.

In der Regel werden getrocknete Reaktanden, Lösungsmittel etc. eingesetzt.

Das Verfahren kann diskontinuierlich oder kontinuierlich, z.B. in einer Rührkesselkaskade, durchgeführt werden. Bevorzugt wird das Verfahren kontinuierlich durchgeführt.

Hierzu werden bei 50°C bis Rückflußtemperatur, bevorzugt bei 65°C bis 85°C, 1 mol/h Anthranilsäureisopropylamid in einem inerten Lösungsmittel, Schwefeltrioxid-Addukt - dargestellt aus 2,0 bis 1,0 mol/h, vorzugsweise 1,4 bis 1,1 mol/h, Schwefeltrioxid und 4,0 bis 1,6 mol/h Base, wie voranstehend beschrieben, in einem inerten Lösungsmittel - und 2,0 bis 0,3 mol/h, vorzugsweise 1,2 bis 0,5 mol/h, Phosphoroxychlorid gleichzeitig in einer mehrstufigen, bevorzugt in einer 2- bis 6-stufigen, insbesondere in einer 3-stufigen Rührkesselkaskade mit einer mittleren Verweilzeit von 6,0 bis 0,5 h, insbesondere 4,0 bis 0,5 Stunden kontinuierlich umgesetzt. Der abgehende "Produktstrom" wird kontinuierlich mit Wasser bei einem pH-Wert von 0 bis 1,5 hydrolysiert. Nach Phasentrennung wird die organische Phase mit einer Base, z.B. Natronlauge, Kalilauge, wäßrige Ammoniaklösung, Diethanolamin, bei pH 6 bis 9 extrahiert. Diese wäßrige Phase wird anschließend kontinuierlich von Resten des organischen Lösungsmittels befreit, z.B. durch Abdestillation. Die resultierende Salzlösung von I kann nun zu den anwendungsorientierten Darreichungsformen weiterverarbeitet werden.

Die Lösung des Schwefeltrioxid-Addukts in inertem Lösungsmittel kann in einem kontinuierlich betriebenen Mischerkreis bei 30 bis 60°C, vorzugsweise bei 35 bis 50°C, hergestellt werden. Hierbei werden kontinuierlich in einem inerten Lösungsmittel gelöste Base, wie voranstehend beschrieben, und Schwefeltrioxid, gegebenenfalls in inertem Lösungsmittel gelöst, zugeführt. Mittels einer Pumpe wird das Reaktionsgemisch intensiv gemischt. Die gebildete Lösung des Schwefeltrioxid-Addukts ist homogen flüssig und wird kontinuierlich abgeführt.

Zur Rückgewinnung der eingesetzten Base wird die bei der Hydrolyse entstehende wäßrige Phase auf pH 10 bis 11 gebracht und mit Lösungsmittel, wie oben aufgeführt, extrahiert. Dieses Base-/Lösungsmittelgemisch wird getrocknet und ggf. durch Zugabe von Base oder Lösungsmittel so eingesetzt, daß es von neuem in das Verfahren eingesetzt werden kann.

Zur Rückgewinnung des Lösungsmittels wird die verbleibende organische Phase ggf. getrocknet und aufdestilliert.

Bevorzugt werden 1 mol/h Anthranilsäureisopropylamid in 1,2-Dichlorethan, 2-Picolin/Schwefeltrioxid-Addukt - hergestellt aus 2,0 bis 1,0 mol/h, insbesondere 1,4 bis 1,1 mol/h, Schwefeltrioxid und 4,0 bis 1,6 mol/h 2-Picolin in 1,2-Dichlorethan - und 2,0 bis 0,3 mol/h, insbesondere 1,2 bis 0,5 mol/h, Phosphoroxychlorid gleichzeitig bei 50°C bis Rückflußtemperatur, bevorzugt bei 65°C bis 85°C, insbesondere bei 70°C bis 85°C, miteinander umgesetzt. Anschließend wird, wie voranstehend beschrieben, zum Produkt hin aufgearbeitet.

Ebenso kann wie im diskontinuierlichen Verfahren anstelle von Schwefeltrioxid Chlorsulfonsäure eingesetzt werden.

Das nach diesem Verfahren herstellbare 3-Isopropyl-1H-2,1,3-benzothiadiazin-4(3H)-on-2,2-dioxid und dessen Salze sind bekannte Pflanzenschutzmittel.

### Herstellbeispiele

### Beispiel 1

Zu 242,0 g einer 18,5%igen Lösung von 2-Picolin in 1,2-Dichlorethan wurden bei 70°C innerhalb von 5 min gleichzeitig 19,2 g Schwefeltrioxid, 285,0 g einer 12,5%igen Lösung von Anthranilsäureisopropylamid in 1,2-Dichlorethan und 30,7 g Phosphoroxychlorid zugetropft. Hierbei stieg die Temperatur auf 80°C an. Nach 3 h Rühren unter Rückfluß wurden 300 ml Wasser zugegeben und die organische Phase abgetrennt. Letztere wurde anschließend mit Natronlauge extrahiert. Nach Ansäuern dieser wäßrigen Phase mit Schwefelsäure wurde mit 1,2-Dichlorethan extrahiert. Die resultierende organische Phase wurde eingeengt.
Ausbeute: 44,7 g 3-Isopropyl-1H-2,1,3-benzothiadiazin-4(3H)-on-2,2-dioxid
(Reinheit: 92,2 %)

### Beispiel 2

Zu 312,0 g einer 18,5%igen Lösung von 2-Picolin in 1,2-Dichlorethan wurden bei 70°C innerhalb von 5 min gleichzeitig 30,0 g Chlorsulfonsäure, 285,0 g einer 12,5%igen Lösung von Anthranilsäureisopropylamid in 1,2-Dichlorethan und 30,7 g Phosphoroxychlorid zugetropft. Nach 3 h Rühren unter Rückfluß wurden 200 ml Wasser zugegeben und die organische Phase abgetrennt. Letztere wurde anschließend mit Natronlauge extrahiert. Nach Ansäuern dieser wäßrigen Phase mit Schwefelsäure wurde mit 1,2-Dichlorethan extrahiert. Die resultierende organische Phase wurde eingeengt.
Ausbeute: 43,3 g 3-Isopropyl-1H-2,1,3-benzothiadiazin-4(3H)-on-2,2-dioxid
(Reinheit: 94,8 %)

### Beispiel 3

Zu 121,0 g einer 18,5%igen Lösung von 2-Picolin in 1,2-Dichlorethan wurde bei Raumtemperatur 19,2 g einer 50%igen Lösung von Schwefeltrioxid in 1,2-Dichlorethan getropft. Dieses Reaktionsgemisch wurde bei 70 bis 80°C gleichzeitig mit 140,0 g einer 12,5%igen Lösung von Anthranilsäureisopropylamid in 1,2-Dichlorethan und 15,3 g Phosphoroxychlorid vereinigt. Nach 3 h Rühren unter Rückfluß kühlte man auf Raumtemperatur ab und versetzte mit 150 ml Wasser. Die organische Phase wurde abgetrennt und mit Natronlauge extrahiert. Die resultierende wäßrige Phase wurde mit Schwefelsäure angesäuert und anschließend mit 1,2-Dichlorethan extrahiert. Letztere wurde dann eingeengt.
Ausbeute: 22,8 g 3-Isopropyl-1H-2,1,3-benzothiadiazin-4(3H)-on-2,2-dioxid
(Reinheit: 89,4 %)

### Beispiel 4

Zu 242,0 g einer 18,5%igen Lösung von 2-Picolin in 1,2-Dichlorethan wurden bei Raumtemperatur 38,4 g einer 50%igen Lösung von Schwefeltrioxid in 1,2-Dichlorethan zugetropft. Bei 70°C wurden anschließend innerhalb von 5 min gleichzeitig 285,0 g einer 12,5%igen Lösung von Anthranilsäureisopropylamid in 1,2-Dichlorethan und 30,7 g Phosphoroxychlorid zugetropft. Hierbei stieg die Temperatur auf 75°C an. Nach 3 h Rühren unter Rückfluß wurden 300 ml Wasser zugegeben und die Phasen getrennt. Die organische Phase wurde mit Natronlauge extrahiert. Die resultierende wäßrige Phase wurde mit Schwefelsäure angesäuert und mit 1,2-Dichlorethan extrahiert. Letztere wurde dann eingeengt.
Ausbeute: 42,8 g 3-Isopropyl-1H-2,1,3-benzothiadiazin-4(3H)-on-2,2-dioxid
(Reinheit: 94,1 %)

### Beispiel 5

Zu 312,0 g einer 18,5%igen Lösung von 2-Picolin in 1,2-Dichlorethan wurden bei Raumtemperatur 30,0 g Chlorsulfonsäure getropft. Bei 70°C wurden anschließend innerhalb von 5 min gleichzeitig 285,0 g einer 12,5%igen Lösung von Anthranilsäureisopropylamid in 1,2-Dichlorethan und 30,7 g Phosphoroxychlorid zugetropft. Hierbei stieg die Temperatur auf 75°C an. Nach 3 h Rühren unter Rückfluß wurden 300 ml Wasser zugegeben und die Phasen getrennt. Die organische Phase wurde mit Natronlauge extrahiert. Die resultierende wäßrige Phase wurde mit Schwefelsäure angesäuert und mit 1,2-Dichlorethan extrahiert. Letztere wurde dann eingeengt.
Ausbeute: 42,4 g 3-Isopropyl-1H-2,1,3-benzothiadiazin-4(3H)-on-2,2-dioxid
(Reinheit: 96,7 %)

### Beispiel 6

Zu 312,0 g einer 18,5%igen Lösung von 2-Picolin in 1,2-Dichlorethan wurden bei 20°C in 15 min 30,0 g Chlorsulfonsäure zugetropft. Bei 70°C wurden anschließend innerhalb von 5 min gleichzeitig 285,0 g einer 12,5%igen Lösung von Anthranilsäureisopropylamid in 1,2-Dichlorethan und 30,7 g Phosphoroxychlorid zugetropft. Nun wurde mit einem vorgeheizten Ölbad der Reaktionsansatz schnell auf Rückfluß erhitzt und 3 h bei dieser Temperatur gerührt. Anschließend wurden 300 ml Wasser zugegeben und die Phasen getrennt. Die organische Phase wurde mit Natronlauge extrahiert, die resultierende wäßrige Phase mit Schwefelsäure angesäuert und mit 1,2-Dichlorethan extrahiert. Letztere wurde dann eingeengt.
Ausbeute: 41,9 g 3-Isopropyl-1H-2,1,3-benzothiadiazin-4(3H)-on-2,2-dioxid
(Reinheit: 96,8 %)

### Beispiel 7

In einer 3-stufigen Rührkolbenkaskade wurden 100 g 1,2-Dichlorethan bei 78°C vorgelegt. Anschließend wurden gleichzeitig bei 78°C 593,6 g/h einer 12,5%igen Lösung von Anthranilsäureisopropylamid (0,417 mol/h) in 1,2-Dichlorethan, 632,3 g/h einer Lösung des 2-Picolin-Schwefeltrioxid-Addukts in 1,2-Dichlorethan (hergestellt aus 589,3 g/h einer 18%igen Lösung von 2-Picolin (1,14 mol/h) in 1,2-Dichlorethan und 43,0 g/h Schwefeltrioxid (0,5375 mol/h)) und 39,9 g/h Phosphoroxychlorid (0,260 mol/h) dem ersten Rührkolben zugeführt, der nach einiger Zeit in den zweiten Rührkolben, der ebenfalls bei 78°C gehalten wurde, überfloß, bis jener den dritten Rührkolben, der ebenfalls auf 78°C gehalten wurde, füllte. Nachdem die Kaskade gefüllt war, wurden für weitere 2 h die Reaktanden zur Umsetzung in den ersten Rührkolben zugeführt. Anschließend wurde die Reaktandenzufuhr abgestellt, der erste Rührkolben 1,5 h, der zweite Rührkolben 1,0 h und der 3. Rührkolben 0,5 h bei 78°C nachgerührt. Anschließend wurde das Reaktionsgemisch mit Wasser hydrolysiert. Es stellte sich ein pH-Wert von 1,0 bis 1,3 ein. Der Inhalt der Rührkolbenkaskade wurde nun kontinuierlich in einen Phasentrenner entleert, die organische Phase kontinuierlich in einen weiteren Rührkolben entleert und mit etwa 150 ml Wasser gewaschen. Nach Phasentrennung wurde die organische Phase in eine 2-stufige Mixer-Settler-Einheit gefördert. In der ersten Stufe wurde mit etwa 150 ml Wasser und 67 g wäßriger Natronlauge extrahiert (hierbei stellte sich ein pH-Wert von 7 bis 8 ein). In der zweiten Stufe wurde die organische Phase mit etwa 50 ml Wasser extrahiert. Die vereinigten wäßrigen Phasen wurden durch Abdestillieren von noch anhaftenden 1,2-Dichlorethan und Wasser aufkonzentriert zu einer etwa 50%igen Lösung.
Ausbeute: 99,4 g Natriumsalz von 3-Isopropyl-1H-2,1,3-benzothia diazin-4(3H)-on-2,2-dioxid

(Die wäßrigen Phasen aus der Hydrolyse wurden mit Natronlauge alkalisch gestellt (ca. pH 10 bis 11) und kontinuierlich mit L,2-Dichlorethan extrahiert. Die entstehende Lösung von 2-Picolin in 1,2-Dichlorethan wurde anschließend durch Azeotoptrocknung im Vakuum (bei ca. 400 mbar) entwässert und eine 2-Picolinkonzentration von ca. 18 % eingestellt. Die so erhaltene Lösung wurde wieder zur Addukt-Bildung eingesetzt).

### Beispiel 8

In einer 3-stufigen Rührkesselkaskade wurden im ersten Rührkessel 100 kg 1,2-Dichlorethan bei 78°C vorgelegt. Anschließend wurden gleichzeitig bei 78°C 593,6 kg/h einer 12,5%igen Lösung von Anthranilsäureisopropylamid (417 mol/h) in 1,2-Dichlorethan, 632,3 kg/h einer Lösung des 2-Picolin-Schwefeltrioxidadduktes in 1,2-Dichlorethan (hergestellt aus 589,3 kg/h einer 18%igen Lösung von 2-Picolin (1140 mol/h) in 1,2-Dichlorethan und 43,0 kg/h Schwefeltrioxid (537,5 mol/h)) und 39,9 kg/h Phosphoroxychlorid (260 mol/h) dem ersten Rührkessel zugeführt, der nach einiger Zeit in den zweiten Rührkessel, der ebenfalls bei 78°C gehalten wurde, überlief, bis jener den dritten Rührkessel, der ebenfalls bei 78°C gehalten wird, füllte. Die Füllstände der Rührkessel wurden so eingestellt, daß die Gesamtverweilzeit zwischen 1,5 und 4 Stunden betrug. Der dritte Rührkessel lief nun kontinuierlich in einen weiteren Rührkessel über, in dem das Reaktionsgemisch mit Wasser bei 50°C bis 70°C hydrolysiert wurde, wobei sich ein pH-Wert von 1 bis 1,4 einstellte. Das zweiphasige Gemisch wurde kontinuierlich getrennt. Die organische Phase wurde kontinuierlich mit Wasser gewaschen, wiederum die Phasen getrennt und anschließend die organische Phase in einer 2-stufigen kontinuierlich getriebenen Mixer-Settler-Einheit in der ersten Stufe mit wäßriger Natronlauge extrahiert (hierbei stellt sich ein pH-Wert von 7 bis 8 ein). In der zweiten Stufe wurde mit wenig Wasser extrahiert. Die vereinigten wäßrigen Phasen wurden durch Abdestillieren von Wasser und anhaftendem 1,2-Dichlorethan zu einer ca. 50%igen Lösung aufkonzentriert.
Ausbeute: 99 bis 100 kg/h Natriumsalz von 3-Isopropyl-1H-2,1,3-benzothiadiazin-4(3H)-on-2,2-dioxid.

(Die wäßrigen Phasen aus der Hydrolyse wurden mit Natronlauge alkalisch gestellt (ca. pH 10 bis 11) und kontinuierlich mit 1,2-Dichlorethan extrahiert. Die entstehende Lösung von 2-Picolin in 1,2-Dichlorethan wurde anschließend durch Azeotroptrocknung im Vakuum (bei ca. 400 mbar) entwässert und eine 2-Picolinkonzentration von ca. 18 % eingestellt. Die so erhaltene Lösung wurde wieder zur Addukt-Bildung eingesetzt.)

## Patentansprüche

1. Verfahren zur Herstellung von 3-Isopropyl-1H-2,1,3-benzothiadiazin-4(3H)-on-2,2-dioxid (I) oder eines Salzes von I 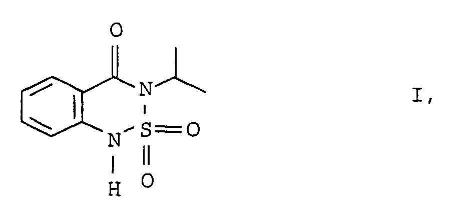 **dadurch kennzeichnet, daß** man gleichzeitig Anthranilsäureisopropylamid II 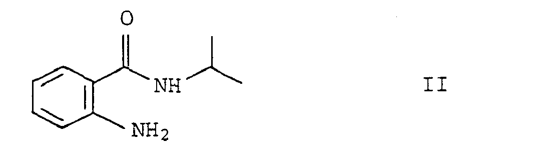 mit Schwefeltrioxid oder Chlorsulfonsäure in Gegenwart einer organischen Base oder mit Addukten von Schwefeltrioxid an organischen Basen
und
Phosphoroxychlorid bei 50°C bis Rückflußtemperatur umsetzt und gewünschtenfalls anschließend in ihre Salze überführt.

2. Verfahren nach Anspruch 1, **dadurch gekennzeichnet, daß** man die Umsetzung in Gegenwart eines Lösungsmittels durchführt.

3. Verfahren nach Anspruch 1 bis 2, **dadurch gekennzeichnet, daß** Anthranilsäureisopropylamid und ggf. das Addukt von Schwefeltrioxid an organischer Base in einem Lösungsmittel zugegeben werden.

4. Verfahren nach Anspruch 1 bis 3, **dadurch gekennzeichnet, daß** man als Lösungsmittel einen halogenierten Kohlenwasserstoff verwendet.

5. Verfahren nach Anspruch 1 bis 4, **dadurch gekennzeichnet, daß** man als Lösungsmittel 1,2-Dichlorethan verwendet.

6. Verfahren nach Anspruch 1 bis 5, **dadurch gekennzeichnet, daß** man als organische Base eine aromatische organische Base verwendet.

7. Verfahren nach Anspruch 1 bis 6, **dadurch gekennzeichnet, daß** man als organische Base Pyridin oder ein substituiertes Pyridin verwendet.

8. Verfahren nach Anspruch 1 bis 7, **dadurch gekennzeichnet, daß** man als organische Base 2-Picolin verwendet.

9. Verfahren nach Anspruch 1 bis 8, **dadurch gekennzeichnet, daß** man die Umsetzung bei 65 bis 85°C durchführt.

10. Verfahren nach Anspruch 1 bis 9, **dadurch gekennzeichnet, daß** man das Verfahren kontinuierlich durchführt.

## Claims

1. A process for preparing 3-isopropyl-1H-2,1,3-benzothiadiazin-4(3H)-one 2,2-dioxide (I) or a salt of I 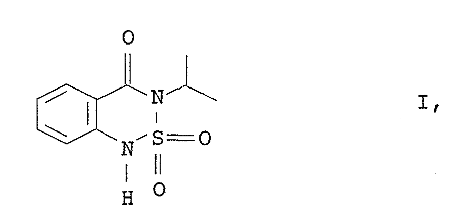 which comprises reacting anthranilic isopropylamide II 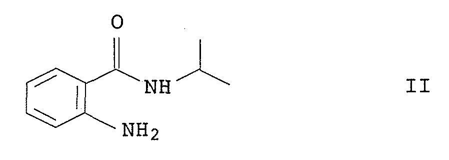 simultaneously with sulfur trioxide or chlorosulfonic acid in the presence of an organic base or with adducts of sulfur trioxide and organic bases
and
phosphorus oxychloride at from 50°C to the reflux temperature, followed, if appropriate, by conversion into its salts.

2. The process according to claim 1, wherein the reaction is carried out in the presence of a solvent.

3. The process according to claim 1 or 2, wherein the anthranilic isopropylamide and, if appropriate, the adduct of sulfur trioxide and organic base are added in a solvent.

4. The process according to any of claims 1 to 3, wherein the solvent used is a halogenated hydrocarbon.

5. The process according to any of claims 1 to 4, wherein the solvent used is 1,2-dichloroethane.

6. The process according to any of claims 1 to 5, wherein the organic base used is an aromatic organic base.

7. The process according to any of claims 1 to 6, wherein the organic base used is pyridine or a substituted pyridine.

8. The process according to any of claims 1 to 7, wherein the organic base used is 2-picoline.

9. The process according to any of claims 1 to 8, wherein the reaction is carried out at from 65 to 85°C.

10. The process according to any of claims 1 to 9, wherein the process is carried out continuously.

## Revendications

1. Procédé de préparation de 2,2-dioxyde de 3-isopropyl-1H-2,1,3-benzothiadiazin-4(3H)-one (I) ou d'un sel de I 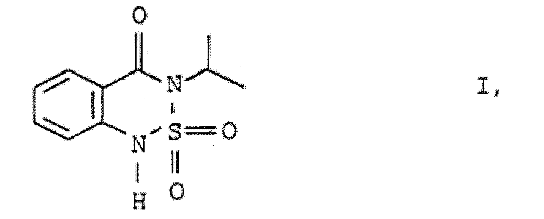 **caractérisé en ce qu'**on fait réagir conjointement de l'isopropylamide d'acide anthranilique II 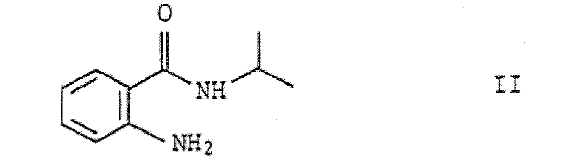 avec du trioxyde de soufre ou de l'acide chlorosulfonique en présence d'une base organique ou avec des produits d'addition de trioxyde de soufre à des bases organiques
et
de l'oxychlorure de phosphore à 50°C jusqu'à la température de reflux, et on effectue ensuite éventuellement une conversion en leurs sels.

2. Procédé suivant la revendication 1, **caractérisé en ce qu'**on effectue la réaction en présence d'un solvant.

3. Procédé suivant la revendication 1 ou 2, **caractérisé en ce que** l'isopropylamide d'acide anthranilique et éventuellement le produit d'addition de trioxyde de soufre à une base organique sont ajoutés dans un solvant.

4. Procédé suivant les revendications 1 à 3, **caractérisé en ce qu'**on utilise un hydrocarbure halogéné comme solvant.

5. Procédé suivant les revendications 1 à 4, **caractérisé en ce qu'**on utilise comme solvant du 1,2-dichloroéthane.

6. Procédé suivant les revendications 1 à 5, **caractérisé en ce qu'**on utilise, comme base organique, une base organique aromatique.

7. Procédé suivant les revendications 1 à 6, **caractérisé en ce qu'**on utilise, comme base organique, de la pyridine ou une pyridine substituée.

8. Procédé suivant les revendications 1 à 7, **caractérisé en ce qu'**on utilise, comme base organique, de la 2-picoline.

9. Procédé suivant les revendications 1 à 8, **caractérisé en ce qu'**on effectue la réaction à 65 jusqu'à 85°C.

10. Procédé suivant les revendications 1 à 9, **caractérisé en ce qu'**on effectue le procédé en continu.
